# EUROPEAN PATENT APPLICATION

(11) **EP 3 591 371 A1**
(43) Date of publication of application: **08.01.2020**
(21) Application number: 18760564.7
(22) Date of filing: 26.02.2018
(51) Int. Cl.: G01N 1/00, G01N 33/497

(54) **OLFACTION TEST DEVICE, OLFACTION TEST METHOD, AND PROGRAM**

(30) Priority: 02.03.2017 JP 2017039789; 22.02.2018 JP 2018029497
(71) Applicant: Penguin System Co., Ltd., Tsukuba-shi, Ibaraki 305-0047 (JP)
(72) Inventor: TAKAGI Daisuke, Tsukuba-shi Ibaraki 305-0047 (JP); KOBAYAKAWA Tatsu, Tsukuba-shi Ibaraki 305-8566 (JP); NIHIRA Takuma, Tsukuba-shi Ibaraki 305-0047 (JP)
(74) Representative: v. Bezold & Partner Patentanwälte - PartG mbB
(86) International application number: PCT/JP2018/006892
(87) International publication number: WO 2018/159518

(57) **Abstract**

An olfaction test device (1) causes air flowing into an inflow path (120) by use of a compressor (121) to have a constant flow velocity and a constant flow rate by passing the air through a sonic nozzle (124). The air is caused to pass through an opened one of inflow valves (126 to 129) disposed in branches of the inflow path (120) and to flow into any one of sample containers (101 to 104), whereby gases with different smell intensities are generated. The gases are then warmed by a heater (153) disposed in an outflow path (150) and released in a nose mask (155).

## Description

### Technical Field

The present disclosure relates to an olfaction test device, an olfaction test method, and a program for testing olfaction by emitting smells.

### Background Art

Known methods for testing human olfaction include emitting smells of different types or intensities and determining whether a test subject can sense such smells. One of the present inventors proposed a gas presentation apparatus in which a first three-way solenoid valve switches connections between a plurality of smell lines and a presentation mask and a second three-way solenoid valve switches connections between a smell-free line and the presentation mask (Patent Literature 1).

While Patent Literature 1 states that a smell bag contains a gas at a desired concentration of a smell component and the gas is introduced into a presentation mask, other known methods include emitting a smell by flowing air into a liquid containing a smell component and by bubbling the liquid with the air (Patent Literature 2). According to explanation in Patent Literature 2, the smell intensity can be varied by changing the bubbling intensity.

### Citation List

### Patent Literature

Patent Literature 1: Unexamined Japanese Patent Application Kokai Publication No. 2011-75389
Patent Literature 2: Unexamined Japanese Patent Application Kokai Publication No. 2013-169234

### Summary of Invention

### Technical Problem

In the gas presentation apparatus according to Patent Literature 1, the air generated at a smell bag is presented to the presentation mask by use of a pneumatic pump, while the outside air is presented to the presentation mask by use of another pneumatic pump. In the method for controlling a smell distribution in a space according to Patent Literature 2, the smell source is bubbled to generate a smell, the small is then mixed with a flow of air generated by a fan, and the mixture is sent out.

These conventional methods involve a change in flow velocity of air caused by switching smell sources or switching to a smell-free air line, and such change in flow velocity may cause the test subject to feel as if the smell has changed. Thus, these methods pose a problem of failing in conducting accurate olfaction tests.

The present disclosure has been made in view of the above-described circumstances, and an objective of the present disclosure is to provide an olfaction test device and others that achieve correct testing without causing test subjects to sense a smell change attributable to other factors.

### Solution to Problem

To achieve the above-described objective, an olfaction test device according to a first aspect of the present disclosure includes:
a sample container that contains a liquid sample constituting a smell source;
an inflow path that causes air having a predetermined flow rate to flow into the sample container at a constant flow velocity;
an inflow valve that passes or shuts off the inflowing air at a point in the inflow path; and
an outflow path that takes in, and flows out toward a test subject, a gas discharged from the sample container by the air inflowing from the inflow path.

The olfaction test device may further include:
a compressor that flows air having at least a certain barometric pressure into the inflow path; and
a flow rate controller that is inserted into the inflow path upstream of the inflow valve.

The olfaction test device may further include:
a compressor that flows air having at least a certain barometric pressure into the inflow path; and
a first branching part that divides an output of the compressor into branches;
flow rate controllers whose number is equal to the number of the branches, the flow rate controllers passing the individual branches of the air divided by the first branching part and having different diameters from one another; and
a first joint that joins together the gas that has been discharged from the sample container by causing at least part of the air passing through the individual flow rate controllers to flow into the sample container and the air that has passed the individual flow rate controllers but has not been caused to flow into the sample container.

The olfaction test device may further include:
a three-way valve including an input end, one output end, and another output end, the input end being connected to an output of the flow rate controller, the one output end being connected to the inflow valve, and the other output end being connected to the outflow path,
wherein the air that has passed through the flow rate controller may be switchable between being directed to the inflow valve side and being directed to the outflow path side.

The olfaction test device may include:
the sample containers, the number of which is two or more, the individual sample containers containing the liquid samples of different types or at different concentrations from one another; and
the inflow valves, the number of which is equal to the number of the sample containers, the individual inflow valves passing or shutting off the air caused to flow into the individual sample containers,
wherein the inflow path may include a second branching part that divides the path into branches upstream of the inflow valves, the number of which branches is equal to the number of the sample containers, and
wherein the outflow path may flow the gas out, the gas having been discharged from one of the sample containers into which the air flows after passing through the inflow valve.

The olfaction test device may further include:
a flush line that flows the air having a flow rate equal to the flow rate of the inflow path; and
a switching valve that switches the air flowing along the flush line between being flowed into the outflow path and being released outside.

The olfaction test device may further include:
a heater that is disposed in the outflow path and warms the gas discharged from the sample container to a temperature falling within a predetermined range.

The outflow path may include a check valve that passes the gas directed from the sample container to the test subject and shuts off the gas directed from the test subject to the sample container.

The olfaction test device may further include:
a filter that removes a smell component from the gas discharged from the sample container; and
a three-way valve that switches the gas discharged from the sample container between being directed to the test subject and being directed to the filter.

An olfaction test method according to a second aspect of the present disclosure is
an olfaction test method for releasing a gas generated from a liquid sample toward a test subject, the liquid sample being contained in a sample container and constituting a smell source, and for determining whether the test subject senses the smell of the gas, the method including:
an inflow step of causing air having a predetermined flow rate to inflow toward the sample container at a constant flow velocity; and
a switching step of switching between discharging and not discharging the gas having a smell from the sample container by switching an inflow valve that passes or shuts off the air that is caused to inflow in the inflow step.

A program according to a third aspect of the present disclosure is
a program executed by a computer for releasing a gas generated from a liquid sample toward a test subject, the liquid sample being contained in a sample container and constituting a smell source, and for determining whether the test subject senses the smell of the gas, the program causing the computer to function as:
an inflow controller that drives a compressor and causes air having a predetermined flow rate to inflow toward the sample container at a constant flow velocity; and
a switcher that switches between discharging and not discharging the gas having a smell from the sample container by switching an inflow valve that passes or shuts off the air that is controlled to inflow by the inflow controller.

### Advantageous Effects of Invention

According to the present disclosure, an accurate test can be conducted without causing the test subject to sense a change in smell based on other factors.

### Brief Description of Drawings

FIG. 1 is a block diagram illustrating a configuration of an olfaction test device according to Embodiment 1;
FIG. 2 illustrates a configuration of a control circuit and peripheral equipment;
FIG. 3 is a flowchart showing an olfaction test process;
FIG. 4 is a block diagram illustrating a configuration of an olfaction test device according to Embodiment 2;
FIG. 5 is a flowchart showing an olfaction test process;
FIG. 6 is a block diagram illustrating a configuration of an olfaction test device according to Embodiment 3;
FIG. 7 shows a relationship among states of three-way valves, states of inflow valves, and gas concentrations;
FIG. 8 is a block diagram illustrating a configuration of an olfaction test device according to Embodiment 4;
FIG. 9 is a block diagram illustrating a configuration of an olfaction test device according to Embodiment 5;
FIG. 10 is a flowchart showing an olfaction test preparation process;
FIG. 11 is a block diagram illustrating a configuration of an olfaction test device according to Embodiment 6;
FIG. 12 is a block diagram illustrating a configuration of an olfaction test device according to Embodiment 7;
FIG. 13 is a flowchart showing an olfaction test preparation process; and
FIG. 14 is a flowchart showing an olfaction test process.

### Description of Embodiments

### (Embodiment 1)

Embodiment 1 of the present disclosure will now be described in detail with reference to the drawings.

An olfaction test device 1 according to the present embodiment flows a gas having a smell toward a test subject to determine whether the test subject senses the smell.

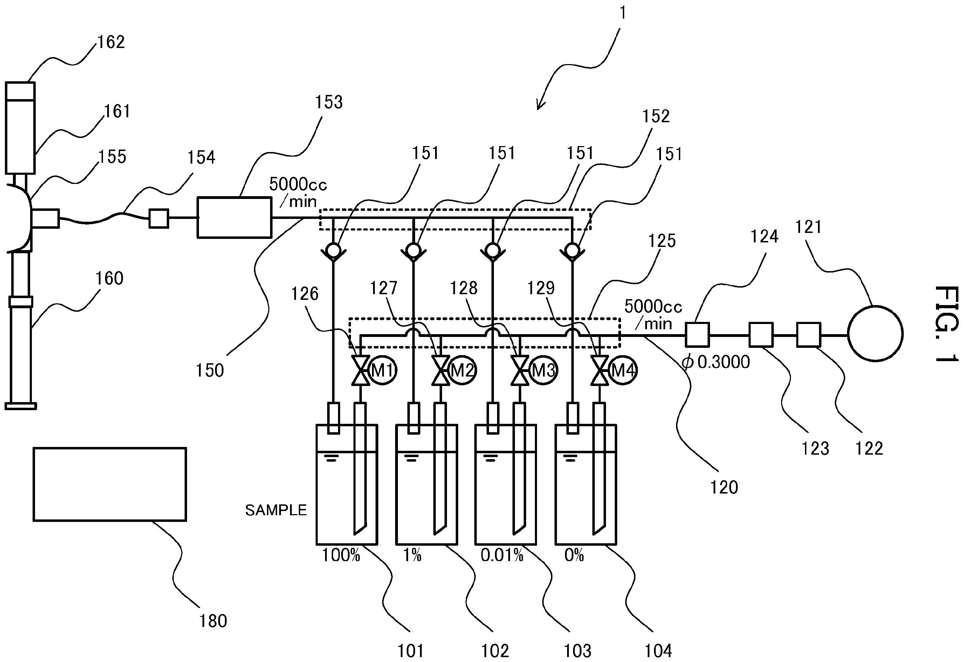

FIG. 1 is a block diagram illustrating a configuration of the olfaction test device 1. As illustrated in FIG. 1, the olfaction test device 1 includes sample containers 101 to 104 that contain liquid samples constituting smell sources, an inflow path 120 that causes air to flow into the liquid sample in each of the sample containers 101 to 104, and an outflow path 150 that takes in a gas generated by bubbling the liquid sample with the air flowing from the inflow path 120 and causes the gas to flow out.

The sample containers 101 to 104 contain the liquid samples having different concentrations. The liquid sample may have any concentration. In the present embodiment, the sample containers 101, 102, 103, and 104 contain the liquid samples having concentrations of 100%, 1%, 0.01%, and 0%, respectively. The liquid sample is a liquid that stimulates an olfactory sense, such as some odorous liquid, and is capable of, when bubbled, generating a gas that has a smell stimulating an olfactory sense.

An exit end of the inflow path 120 is located near the bottom of the liquid sample in each of the sample containers 101 to 104, while an entry end of the outflow path 150 is located above the liquid level of each liquid sample. The sample containers 101 to 104 are each sealed except the inflow path 120 and the outflow path 150 so as not to leak smells outside.

A compressor 121 is connected to an entry end of the inflow path 120. The inflow path 120 includes a pressure reducing valve 122, a filter 123, a sonic nozzle 124, a branching part 125, and inflow valves (M1 to M4) 126 to 129, in the order mentioned along the air flow direction. The air that has been output from the compressor 121 undergoes pressure reduction to a predetermined pressure not lower than a certain value in the pressure reducing valve 122, and odorous components are removed by the filter 123 to make the air smell-free.

The sonic nozzle 124 maintains the flow velocity of the air flowing out of the sonic nozzle 124 at the speed of sound by keeping the pressure ratio between upstream and downstream not higher than the critical pressure ratio. In the illustrated example, the pressure ratio between upstream and upstream (downstream/upstream) is kept not higher than the critical pressure ratio by reducing the air pressure to at least a certain value through the pressure reducing valve 122. The sonic nozzle 124 flows out the air having a constant flow rate that depends on the diameter thereof. The flow rate of the air may be determined as appropriate according to test details. In the present embodiment, the sonic nozzle 124 has a diameter of 0.3 mm to flow out the air having a flow rate of 5,000 cc/min.

The inflow path 120 is divided into four branches at the branching part 125 located downstream of the sonic nozzle 124. The four branches of the inflow path 120 divided at the branching part 125 respectively include the inflow valves (M1 to M4) 126 to 129 and the exit ends. Each of the inflow valves (M1 to M4) 126 to 129 includes a two-way solenoid valve and is capable of opening and closing the valve in accordance with an externally input control signal to switch between passing and shutting off a flow.

Each of the exit ends of the branched inflow path 120 is positioned near the bottom of each of the sample containers 101 to 104. Thus, the air passes through the sonic nozzle 124, divided into four branches at the branching part 125, passes through any of the inflow valves (M1 to M4) 126 to 129 that is open, and is released into the liquid sample in any of the sample containers 101 to 104.

The outflow path 150 includes four entry ends disposed in the sample containers 101 to 104 to take in a gas above the liquid level of each of the sample containers 101 to 104. The outflow path 150 includes a check valve 151, a joint 152, a heater 153, and a flexible hose 154 in the order mentioned along the gas flow direction. The outflow path 150 at its exit end is connected to a nose mask 155.

The check valve 151 disposed in each of the four outflow paths 150 leaving the sample containers 101 to 104 allows a gas to flow from the sample containers 101 to 104 to the heater 153 only, preventing the gas from flowing in the opposite direction. This avoids causing a gas flowing out of one container to flow into another container to be mixed with another gas.

The four outflow paths 150 join together in the joint 152 located downstream of the check valves 151. After the outflow paths join together, the gas has a flow rate of 5,000 cc/min, which is equal to that of the inflow path 120. The outflow path 150 has a heater 153 disposed downstream of the joint 152. Since a smell is sensed differently depending on the temperature, the heater 153 is used to control the gas passing therethrough to have a temperature falling within a predetermined range.

The flexible hose 154, which is an air tube having flexibility, is made of Teflon®, for example. The nose mask 155 can be placed at a desired position by bending the flexible hose 154 freely. The nose mask 155 is supported by a supporting platform 160. The supporting platform 160 includes a lifting and lowering mechanism and is capable of moving the nose mask 155 vertically.

The nose mask 155 is in a substantially hemispherical shape so as to cover the nose of a test subject. In the center of the nose mask 155, there is provided a vent hole to which the exit end of the outflow path 150 is connected. On top of the nose mask 155, there is provided another vent hole that is connected to an air passage equipped with an activated carbon filter 161 and a fan 162.

The fan 162 rotates to move a gas from the nose mask 155 to the outside. In addition, the fan 162 produces a noise when rotating. The noise drowns out a switching sound produced by, for example, the inflow valves (M1 to M4) 126 to 129, thereby avoiding causing the test subject to detect, based on such switching sound or the like, that smells have been changed.

The activated carbon filter 161 removes smell components from the gas passing therethrough. In this way, the gas in the nose mask 155 can be made smell-free when released outside.

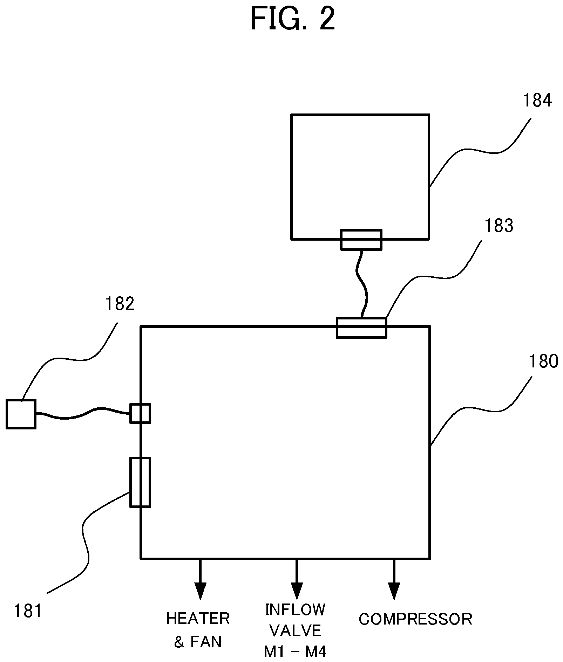

The compressor 121, the heater 153, the fan 162, and the inflow valves (M1 to M4) 126 to 129 are electrically connected to a control circuit 180. FIG. 2 illustrates a configuration of the control circuit 180 and peripheral equipment. The control circuit 180 controls power supply to the compressor 121, the heater 153, and the fan 162 while controlling the inflow valves (M1 to M4) 126 to 129 to switch between passing and shutting off a flow.

The control circuit 180 includes a main power switch 181, a sense switch 182, and a USB connector 183. The main power switch 181 turns on/off overall the power to the control circuit 180 and the power supplied to other components of the olfaction test device 1 via the control circuit 180. The sense switch 182 is pressed by the test subject when a smell is sensed.

An information terminal 184 is connected to the USB connector 183. The information terminal 184 is a computer equipped with a central processing unit (CPU) and memory and is capable of USB communications/connections. The information terminal 184 may be, for example, a tablet terminal or a smart phone. The information terminal 184 has an olfaction test processing program installed thereon to send control signals to the control circuit 180 when the program is executed. On the basis of control signals input from the information terminal 184, the control circuit 180 controls power supply to the compressor 121, the heater 153, and the fan 162 and controls the inflow valves (M1 to M4) 126 to 129 to switch between opening and closing the valves.

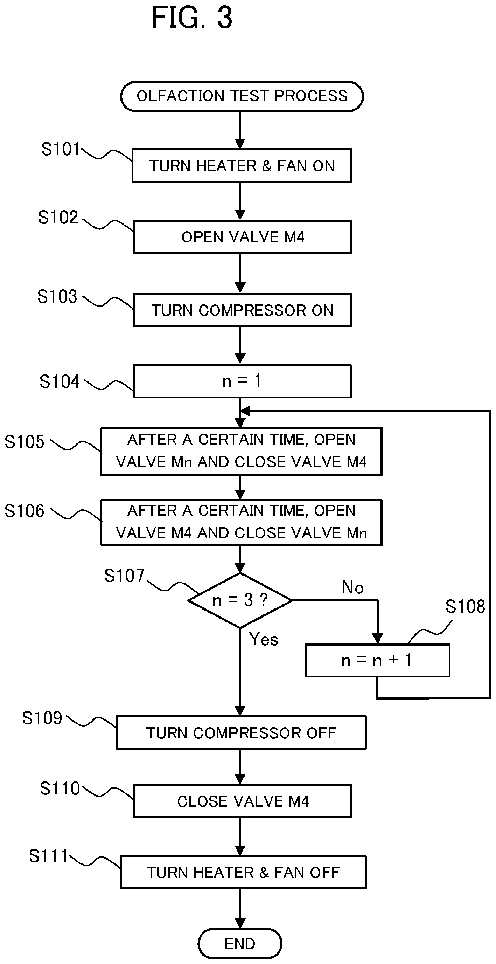

Operations of the olfaction test device 1 as configured above will now be described in detail with reference to the flowchart in FIG. 3. FIG. 3 is a flowchart showing an olfaction test process.

The olfaction test process starts when the main power switch 181 is turned on and the information terminal 184 executes the olfaction test program. All the inflow valves (M1 to M4) 126 to 129 are closed at this time.

First, the control circuit 180 supplies power to, and starts driving, the heater 153 and the fan 162 (step S101). Next, the control circuit 180 opens the inflow valve (M4) 129 (step S102) and starts driving the compressor 121 (step S103). As the compressor 121 is driven, air starts flowing into the inflow path 120.

The air flowing from the compressor 121 undergoes pressure reduction to a predetermined pressure in the pressure reducing valve 122 and is made smell-free through the filter 123. Then, the air passes through the sonic nozzle 124 having a diameter of 0.3000 mm. Since the air passes through the sonic nozzle 124 at a flow velocity equal to the speed of sound, the flow rate of the air becomes 5,000 cc/min.

As the inflow valve (M4) 129 was opened in step S102, the air flowing out of the sonic nozzle 124 is released in the liquid sample in the sample container 104 and the liquid sample is bubbled. The liquid sample in the sample container 104 contains a 0% concentration of a smell component. Thus, the gas that is produced through bubbling, taken from the sample container 104 into the outflow path 150 and passed through the outflow path 150, and released in the nose mask 155 has no smell.

After released in the nose mask 155, the air passes through the activated carbon filter 161 and is released to the outside by a suction force created by the fan 162.

Upon start of the olfaction test, n is set to 1 (n = 1) (step S104). After a predetermined time has passed, the inflow valve (M1) 126 is opened and the inflow valve (M4) 129 is closed (step S 105). As a result, the air flowing out of the sonic nozzle 124 passes through the inflow valve (M1) (126) to be released in the liquid sample in the sample container 101, and the liquid sample is bubbled.

The liquid sample in the sample container 104 contains a 100% concentration of a smell component. Thus, the gas that is produced through bubbling and taken from the sample container 104 into the outflow path 150 has a smell with an intensity corresponding to a concentration of 100%. Upon sensing the smell, the test subject presses the sense switch 182. The control circuit 180 sends to the information terminal 184 a signal indicating that the sense switch 182 has been pressed, and the information terminal 184 stores the result indicating that a smell has been sensed.

Note that the gas is warmed by the heater 153 to a temperature falling within a predetermined range before released into the nose mask 155 because a smell is sensed differently depending on the temperature. Also note that, as described above, the gas produced through bubbling has a constant flow velocity and a constant flow rate because the flow velocity and flow rate of the air flowing out of the sonic nozzle 124 are kept at a constant level. Also note that the gas flows without interruption because the inflow valve (M1) 126 is opened and then the inflow valve (M4) 129 is closed. Therefore, the test subject is prevented from determining, attributable to a change in flow velocity or flow rate of the gas, that the smell has changed.

When a predetermined time has passed after opening of the inflow valve (M1) 126, the inflow valve (M4) 129 is opened and the inflow valve (M1) 126 is closed (step S106). The sample in the sample container 104 starts bubbling, and the smell-free air goes through the outflow path 150 to be released from the nose mask 155.

Note that the air, after released into the nose mask 155, passes through the nose mask 155, and is made smell-free in the activated carbon filter 161 and released to the outside because the fan 162 keeps rotating while the power is on. Thus, the air with a smell no longer stays in the nose mask 155 and, accordingly, the test subject can also recognize that the air becomes smell-free. The fan 162 outputs a noise of a certain level. The noise output by the fan 162 that keeps on rotating drowns out switching sounds made by the inflow valves (M1 to M4) 126 to 129. Therefore, the test subject is prevented from determining, on the basis of the switching sound, that the smell has changed.

Then, it is determined that n is not 3 (No in step S107), n is incremented by 1 (step S108), and the processing returns to step S105. Steps S105 to S108 are repeated thereafter.

When it is determined that n is 3 in step S 107 (Yes in step S107), which means the test with the liquid samples at 100%, 1%, and 0.01% concentrations is finished, the compressor 121 is turned off (step S109). Next, the inflow valve (M4) 129 is closed (step S110), the heater 153 and the fan 162 being driven are stopped (step S111), and the process is exited.

In this way, the air having a constant flow velocity and a constant flow rate is caused to flow out of the sonic nozzle 124 into the four sample containers 101 to 104 successively, whereby gases with different smell intensities are released from the nose mask 155. The test subject inhales gases with different smell intensities at regular time intervals to be tested whether the test subject can sense the smells.

As described above, the olfaction test device 1 according to the present embodiment causes the air flowing into the inflow path 120 by use of the compressor 121 to have a constant flow velocity and a constant flow rate by passing the air through the sonic nozzle 124. Next, the air is caused to pass through any opened one of the inflow valves (M1 to M4) 126 to 129 disposed in the branched inflow paths 120 and flow into the liquid sample in one of the sample containers (101 to 104). The liquid sample is then bubbled to generate a gas with different smell intensities. The gas is then warmed by the heater 153 disposed in the outflow path 150 and is released in the nose mask 155. As a result, an accurate test can be conducted without causing the test subject to sense a change in smell based on other factors including a change in flow velocity or flow rate.

### (Embodiment 2)

Embodiment 2 of the present disclosure will now be described in detail with reference to the drawings.

As in Embodiment 1, an olfaction test device 2 according to the present embodiment flows a gas having a smell toward a test subject to determine whether the test subject senses the smell.

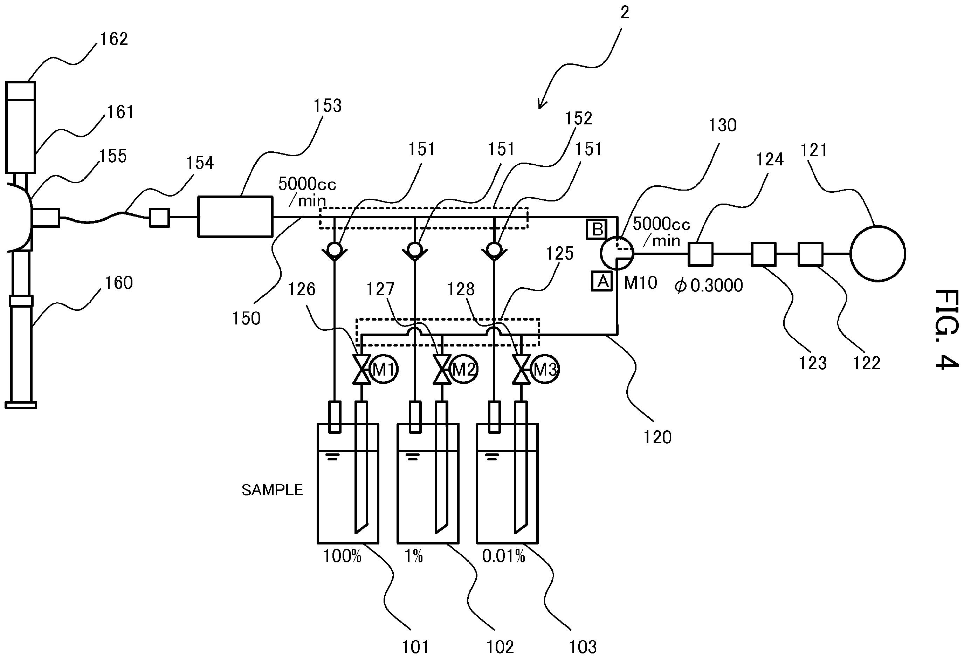

FIG. 4 is a block diagram illustrating a configuration of the olfaction test device 2 according to the present embodiment. As illustrated in FIG. 4, the olfaction test device 2 includes sample containers 101 to 103 that contain liquid samples constituting smell sources, an inflow path 120 that causes air to flow into the liquid sample in each of the sample containers 101 to 103, and an outflow path 150 that takes in a gas generated by bubbling the liquid sample with the air flowing from the inflow path 120 and causes the gas to flow out.

The present embodiment is the same as Embodiment 1 regarding the sample containers 101 to 103, the inflow valves (M1 to M3) 126 to 128, the configuration of the inflow path 120 from the compressor 121 to the sonic nozzle 124, and the outflow path 150 from the heater 153 to the end. The present embodiment is different from Embodiment 1 in that neither the sample container 104 nor the inflow valve (M4) 129 is included and that a three-way valve (M10) 130 is disposed downstream of the sonic nozzle 124. The configuration of the control circuit 180 and its peripheral is also the same as in Embodiment 1, but the olfaction test process executed by the information terminal 184 is partly different.

The three-way valve (M10) 130 switches the passing direction of the air flowing out of the sonic nozzle 124 between the sample containers 101 to 103 side (direction A) and the outflow path 150 side (direction B). The three-way valve (M10) 130, which includes a solenoid valve, is controlled to switch directions by control signals input from the control circuit 180.

When the three-way valve (M10) 130 is connected to the direction A, the air flowing out of the sonic nozzle 124 passes through any opened one of the inflow valves (M1 to M3) 126 to 128 and flows into one of the sample containers 101 to 103. The liquid sample in the sample containers 101 to 103 into which the air has inflowed is bubbled. When the three-way valve (M10) 130 is connected to the direction B, the air flowing out of the sonic nozzle 124 directly passes through the joint 152, is warmed by the heater 153, and is released from the nose mask 155.

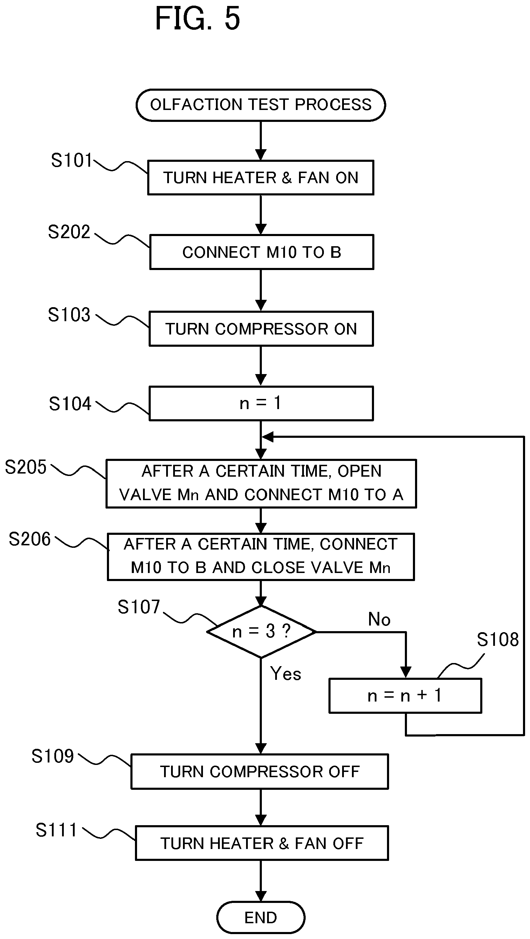

Operations of the olfaction test device 2 as configured above will now be described in detail with reference to the flowchart in FIG. 5. FIG. 5 is a flowchart showing an olfaction test process according to the present embodiment.

The olfaction test process starts when the main power switch 181 is turned on and the information terminal 184 executes the olfaction test program. All the inflow valves (M1 to M3) 126 to 128 are closed at this time.

First, the control circuit 180 supplies power to, and starts driving, the heater 153 and the fan 162 (step S101). Next, the control circuit 180 connects the three-way valve (M10) 130 to the direction B (step S202), and then starts driving the compressor 121 (step S103). As the compressor 121 is driven, air starts flowing into the inflow path 120.

The air flowing from the compressor 121 undergoes pressure reduction to a predetermined pressure in the pressure reducing valve 122 and is made smell-free through the filter 123. Then, the air passes through the sonic nozzle 124 having a diameter of 0.3000 mm. Since the air passes through the sonic nozzle 124 at a flow velocity equal to the speed of sound, the flow rate of the air becomes 5,000 cc/min.

Since the three-way valve (M10) 130 was connected to the direction B in step S202, the air flowing out of the sonic nozzle 124 passes through the joint 152, the heater 153, and the flexible hose 154, and is released from the nose mask 155. At this time, the air released from the nose mask 155 is smell-free.

Upon start of the olfaction test, n is set to 1 (n = 1) (step S104). After a predetermined time has passed, the inflow valve (M1) 126 is opened and the three-way valve (M10) 130 is connected to the direction A (step S205). As a result, the air flowing out of the sonic nozzle 124 flows through the three-way valve (M10) 130 directed to the direction A and passes through the inflow valve (M1) (126) to be released in the liquid sample in the sample container 101, and the liquid sample is bubbled.

The liquid sample in the sample container 104 contains a 100% concentration of a smell component. Thus, the air that is produced through bubbling and taken from the sample container 104 into the outflow path 150 has a smell with an intensity corresponding to a concentration of 100%. Upon sensing the smell, the test subject presses the sense switch 182. The control circuit 180 sends to the information terminal 184 a signal indicating that the sense switch 182 has been pressed, and the information terminal 184 stores the result indicating that a smell has been sensed.

Note that the gas produced through bubbling has a constant flow velocity and a constant flow rate because the flow velocity and flow rate of the air flowing out of the sonic nozzle 124 are kept at a constant level. Also note that the gas flows without interruption because the inflow valve (M1) 126 is opened and then the three-way valve (M10) 130 is connected to the direction A. Therefore, the test subject is prevented from determining, attributable to a change in flow velocity or flow rate of the gas, that the smell has changed.

When a predetermined time has passed after opening of the inflow valve (M1) 126, the three-way valve (M10) 130 is connected to the direction B, and then the inflow valve (M1) 126 is closed (step S206). Thus, the smell-free air flowing out of the sonic nozzle 124 passes through the joint 152, the heater 153, and the flexible hose 154, and is released from the nose mask 155.

The air, after released into the nose mask 155, passes through the nose mask 155, and is made smell-free in the activated carbon filter 161 and released to the outside because the fan 162 keeps rotating while the power is on. Thus, the air with a smell no longer stays in the nose mask 155 and, accordingly, the test subject can also recognize that the air becomes smell-free. The fan 162 outputs a noise of a certain level. The noise output by the fan 162 that keeps on rotating drowns out switching sounds made by the inflow valves (M1 to M3) 126 to 128 and the three-way valve (M10) 130. Therefore, the test subject is prevented from determining, on the basis of the switching sound, that the smell has changed.

Then, it is determined that n is not 3 (No in step S107), n is incremented by 1 (step S108), and the processing returns to step S205. Steps S205, S206, S107, and S108 are repeated thereafter.

When it is determined that n is 3 in step S 107 (Yes in step S107), which means the test with the liquid samples at 100%, 1%, and 0.01% concentrations is finished, the compressor 121 is turned off (step S109). Next, the heater 153 and the fan 162 being driven are stopped (step S111), and the process is exited.

In this way, the olfaction test device 2 uses the three-way valve (M10) 130 to alternately switch the air flow directions so that the smell-free air is directly released into the nose mask and then the air having one of different smell intensities is produced by bubbling the air in one of the three sample containers 101 to 103 in this order and released. The test subject inhales the air with different smell intensities at regular time intervals to be tested whether the test subject can sense the smells.

As described above, the olfaction test device 2 according to the present embodiment causes the air flowing out of the compressor 121 to have a constant flow velocity and a constant flow rate through the sonic nozzle 124, and switches the air flow directions through the three-way valve (M10) 130. Sending the air directly to the nose mask 155 at regular intervals and sending the air having a smell to the nose mask 155 are repeated in an alternate manner, where the air having a smell is produced by causing the air to flow into the liquid sample in each of the sample containers 101 to 103 and by bubbling the liquid sample with the air. The present embodiment simplifies a configuration for sending the smell-free air while achieving an accurate test in which the test subject can hardly recognize a change in flow velocity and the like.

### (Embodiment 3)

Embodiment 3 of the present disclosure will now be described in detail with reference to the drawings.

As in Embodiments 1 and 2, an olfaction test device 3 according to the present embodiment flows a gas having a smell toward a test subject to determine whether the test subject senses the smell.

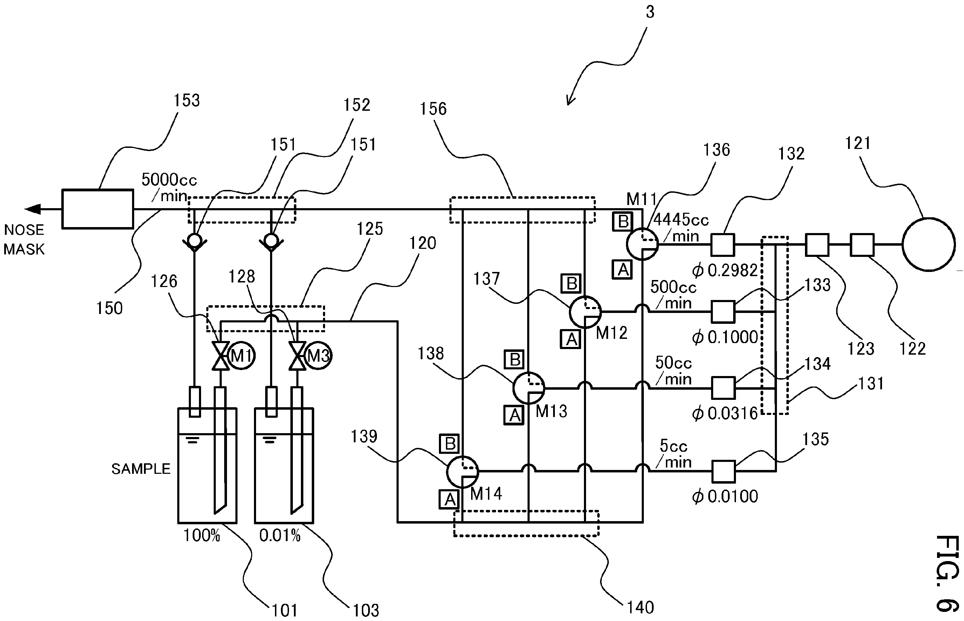

FIG. 6 is a block diagram illustrating a configuration of the olfaction test device 3 according to the present embodiment. As illustrated in FIG. 6, the olfaction test device 3 includes sample containers 101 and 103 that contain liquid samples constituting smell sources, an inflow path 120 that causes air to flow into the liquid sample in each of the sample containers 101 and 103, and an outflow path 150 that takes in a gas generated by bubbling the liquid sample with the air flowing from the inflow path 120 and causes the gas to flow out.

The present embodiment is the same as Embodiment 1 regarding the sample containers 101 and 103, the inflow valves (M1 and M3) 126 and 128, the configuration of the inflow path 120 from the compressor 121 to the filter 123, and the outflow path 150 from the heater 153 to the end. The present embodiment is different from Embodiment 1 in that a plurality of sonic nozzles and a plurality of three-way valves are included. The configuration of the control circuit 180 and its peripheral is also the same as in Embodiment 1, but the olfaction test process executed by the information terminal 184 is partly different.

The branching part 131 (a first branching part) is disposed downstream of the filter 123, and sonic nozzles 132 to 135 having different diameters are disposed in the paths branched by the branching part 131. The flow velocity of the air flowing out of each of the sonic nozzles 132 to 135 is equal to the speed of sound. The air flow rate may be determined as appropriate according to test details. The present embodiment is described with the assumption that the air has the following flow rates. The sonic nozzle 132 has a diameter of 0.2982 mm to provide a flow rate of 4,445 cc/min. The sonic nozzle 133 has a diameter of 0.1000 mm to provide a flow rate of 500 cc/min. The sonic nozzle 134 has a diameter of 0.0316 mm to provide a flow rate of 50 cc/min. The sonic nozzle 135 has a diameter of 0.0100 mm to provide a flow rate of 5 cc/min. Thus, the sonic nozzles 132 to 135 provide a total flow rate of 5,000 cc/min.

The three-way valves (M11 to M14) 136 to 139 are disposed downstream of their corresponding sonic nozzles 132 to 135. Input ends of the three-way valves (M11 to M14) 136 to 139 are connected to output ends of the corresponding sonic nozzles 132 to 135. One output end of each of the three-way valves (M11 to M14) 136 to 139 is connected to the inflow valves (M1 and M3) 126 and 128 while the other output end is connected to the outflow path 150. The three-way valves (M11 to M14) 136 to 139 each switch the passing direction of the air flowing out of the sonic nozzles 132 to 135 between the sample containers 101 and 103 side (direction A) and the outflow path 150 side (direction B). The three-way valves (M11 to M14) 136 to 139, each of which includes a solenoid valve, are controlled to switch directions by control signals input from the control circuit 180.

The joint 140 is disposed downstream of the three-way valves (M11 to M14) 136 to 139 on the direction A side and upstream of the branching part 125 (a second branching part). The joint 156 is disposed downstream of the three-way valves (M11 to M14) 136 to 139 on the direction B side and upstream of the joint 152 (a first joint).

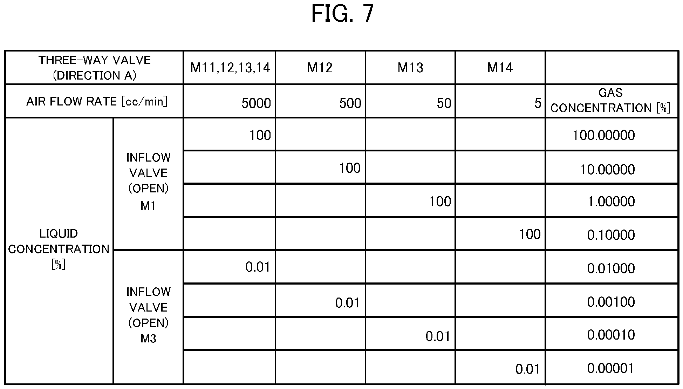

The air with a variable smell intensity can be released from the nose mask 155 by switching the three-way valves (M11 to M14) 136 to 139, the inflow valve (M1) 126, and the inflow valve (M3) 128. The following describes how the smell intensity is changed referring to FIG. 7. FIG. 7 shows a relationship among states of the three-way valves (M11 to M14) 136 to 139, states of the inflow valve (M1) 126 and the inflow valve (M3) 128, and gas concentrations.

As shown in FIG. 7, when all the three-way valves (M11 to M14) 136 to 139 are connected to the direction A and the inflow valve (M1) 126 is opened, the air flows at 5,000 cc/min into the 100% sample container 101 to be bubbled.

The gas flowing out of the sample container 101 then passes through the joint 152, is warmed in the heater 153, and is released from the nose mask 155. At this time, the gas has a concentration of substantially 100% and a flow rate of 5,000 cc/min.

In another example, when the three-way valve (M12) 137 among the three-way valves (M11 to M14) 136 to 139 is connected to the direction A with the other three-way valves connected to the direction B, and the inflow valve (M1) 126 is opened, the air flows at 500 cc/min into the 100% sample container 101 to be bubbled. The smell-free air from the other three-way valves (M11, M13, and M14) 136, 138, and 139 flows in the direction B and directly combined together in the joint 156.

Then, the gas taken into the outflow path 150 from the sample container 101 and the gas combined in the joint 156 join together in the joint 152, and the resulting gas is warmed in the heater 153 and released from the nose mask 155. The gas concentration is now substantially 10% and the gas flow rate is 5,000 cc/min.

In still another example, when the three-way valve (M12) 137 among the three-way valves (M11 to M14) 136 to 139 is connected to the direction A with the other three-way valves connected to the direction B, and the inflow valve (M3) 128 is opened, the air flows at 500 cc/min into the 0.01% sample container 103 to be bubbled. The smell-free air from the other three-way valves (M11, M13, and M14) 136, 138, and 139 flows in the direction B and directly combined together in the joint 156.

Then, the gas taken into the outflow path 150 from the sample container 103 and the gas combined in the joint 156 join together in the joint 152, and the resulting gas is warmed in the heater 153 and released from the nose mask 155. The gas concentration is now substantially 0.001% and the gas flow rate is 5,000 cc/min.

In this way, the gas having a substantial concentration ranging from 100% to 0.00001% and flowing at a constant flow rate of 5,000 cc/min is achieved by switching the three-way valves and inflow valves in accordance with the table in FIG. 7.

In addition, the smell-free gas having a concentration of 0% and a flow rate of 5,000 cc/min can be released from the nose mask 155 by connecting all the three-way valves (M11 to M14) 136 to 139 to the direction B.

Operations of the olfaction test device 3 configured as above are different from those in Embodiment 1 in the process of bubbling a liquid sample. Specifically, instead of carrying out steps S104 to S108 in the olfaction test process illustrated in FIG. 3, the olfaction test device 3 performs the process of releasing the gas having a variable concentration and releasing the smell-free air in an alternate manner by changing the flow rate of the air flowing into the sample container 101 or 103 in accordance with the table in FIG. 7. As seen above, gases of various concentrations can be released, which makes it possible to find at which gas concentration level, or smell intensity, the test subject can sense a smell.

As described above, the olfaction test device 3 according to the present embodiment causes the air flowing from the compressor 121 to divide into branches in the branching part 131 and to pass through the sonic nozzles 132 to 135 having different diameters. Then, the air passes through the three-way valves (M11 to M14) 136 to 139 disposed downstream of their corresponding sonic nozzles 132 to 135 to be either directed to the sample containers 101 and 103 or directly flowed into the nose mask 155, thereby producing gases in varying concentrations and accordingly with varying smell intensities. As a result, various gas concentration levels can be set to achieve a more accurate olfaction test.

### (Embodiment 4)

Embodiment 4 of the present disclosure will now be described in detail with reference to the drawings.

As in Embodiments 1 to 3, an olfaction test device 4 according to the present embodiment flows a gas having a smell toward a test subject to determine whether the test subject senses the smell.

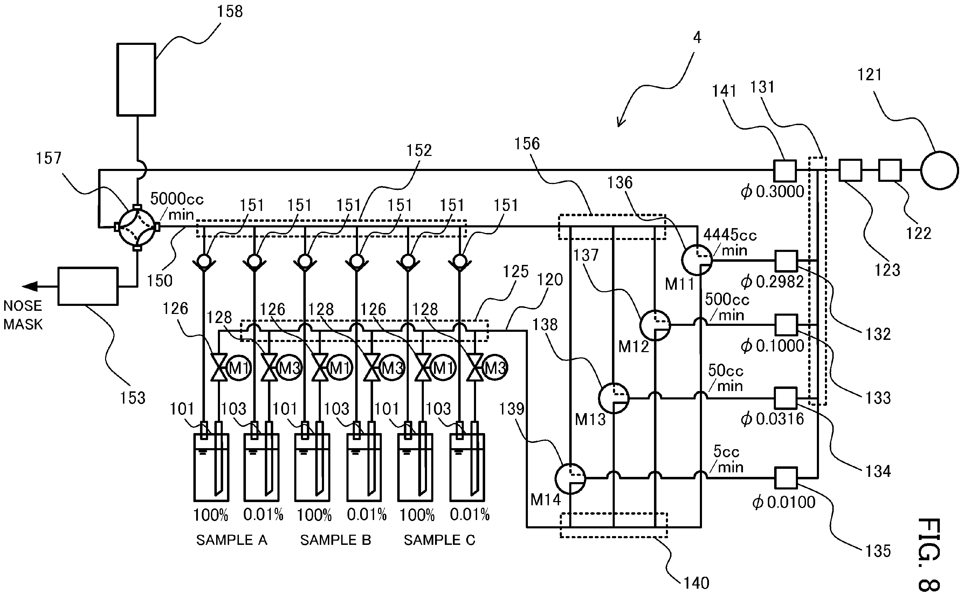

FIG. 8 is a block diagram illustrating a configuration of the olfaction test device 4 according to the present embodiment. As illustrated in FIG. 8, the olfaction test device 4 includes a plurality of pairs of sample containers 101 and 103 containing liquid samples constituting smell sources, where the type of liquid sample differs among the pairs. The olfaction test device 4 further includes an inflow path 120 that causes air to flow into the liquid sample in each of the sample containers 101 and 103, and an outflow path 150 that takes in a gas generated by bubbling the liquid sample with the air flowing from the inflow path 120 and causes the gas to flow out.

Components of the olfaction test device are the same as those in Embodiment 3 except that the olfaction test device includes a plurality of inflow paths 120 inserted into the liquid samples of different types from the branching part 125. In the present embodiment, the liquid samples in concentrations of 100% and 0.01% are contained in the sample containers 101 and 103 for each of the samples A, B, and C and, in the branching part 125, the inflow path 120 is divided into six branches whose exit ends are located in the individual liquid samples.

When the air flowing out of the four sonic nozzles 132 to 135, which are used in Embodiment 3, is directed to the direction A in the three-way valves (M11 to M14) 136 to 139, the air is combined in the joint 140. The combined air then passes through one opened valve among the six inflow valves 126 and 128 opposed to the sample containers containing the samples A, B, and C, and the corresponding liquid sample is bubbled with the air.

As in Embodiment 3, the air used for bubbling has a flow rate selected from the four different flow rates. Thus, the three types of liquid samples each having two concentration levels can be bubbled with the air flowing at four different flow rates, and accordingly, three types of smells each having eight different intensities can be produced.

In the present embodiment, which uses different types of smells, a flush line is provided in order to avoid mixing these smells. Specifically, as illustrated in FIG. 8, an extra branch is added so that the path is divided into five branches in the branching part 131, which is connected to a sonic nozzle 141 having a diameter of 0.3000 mm in addition to the four sonic nozzles 132 to 135 as in Embodiment 3.

In this example, the air from the four sonic nozzles 132 to 135 flows at a total flow rate of 5,000 cc/min while the air from the sonic nozzle 141 flows at a flow rate of 5,000 cc/min. Thus, the air introduced from the compressor 121 flows at a total flow rate of 10,000 cc/min.

A four-way valve 157 is disposed between the joint 152 and the heater 153, and the remaining two terminals of the valve are connected to the sonic nozzle 141 and to the activated carbon filter 158, respectively. When connections of the four-way valve 157 allow a flow to pass from the sonic nozzle 141 through the activated carbon filter 158, a flow is allowed to pass from the joint 152 through the heater 153. In this case, the olfaction test device 4 is operating such that the different types of liquid samples are bubbled at different gas concentrations, and the gases with different smell intensities are warmed in the heater 153 and released from the nose mask 155.

When connections of the four-way valve 157 allow a flow to pass from the sonic nozzle 141 through the heater 153, a flow is allowed to pass from the joint 152 through the activated carbon filter 158. In this case, the olfaction test device 4 is operating such that smell-free air from the sonic nozzle 141 passes through the four-way valve 157 and then passes through the heater 153, the flexible hose 154, and the nose mask 155. In this way, the heater 153, the flexible hose 154, and the nose mask 155 can be cleaned with the smell-free air so that no smell remains therein. During the cleaning, the gas flowing out of the joint 152 passes through the four-way valve 157 and the activated carbon filter 158 to become smell-free and released into the outside air.

As seen above, the flush line including the sonic nozzle 141 and the four-way valve 157 is disposed, which makes it possible to clean the heater 153 and others with the flush line when the different types of liquid samples are being bubbled. As a result, mixing different smells from different types of liquid samples can be avoided. In this way, the four-way valve 157 functions as a switching valve that switches between directing the air flowing through the flush line to the outflow path 150 with the heater 153 and the nose mask 155 disposed and directing the air to the activated carbon filter 158 so that the air passing through the activated carbon filter is released to the outside.

As described above, in the present embodiment, different types of smells each having a plurality of smell intensities are generated by causing air to flow at a predetermined flow rate through the sonic nozzles 132 to 135 and the three-way valves (M11 to M14) 136 to 139 into the sample containers 101, 103 containing different types of liquid samples and by bubbling the samples with the air. In addition, when the samples are not bubbled, the air passing through the sonic nozzle 141 is caused to pass through the heater 153, the flexible hose 154, and the nose mask 155 via the four-way valve 157. As a result, different types of smells each having a plurality of intensities can be released from the nose mask 155 while the nose mask 155 can be cleaned with smell-free air, thereby achieving an accurate olfaction test including testing of the sense of different types of smells.

### (Embodiment 5)

Embodiment 5 of the present disclosure will now be described in detail with reference to the drawings.

As in Embodiments 1 to 4, an olfaction test device 5 according to the present embodiment flows a gas having a smell toward a test subject to determine whether the test subject senses the smell.

There are two major differences between the olfaction test device 5 according to the present embodiment and those according to Embodiments 1 to 4. One is that the sample container is filled with a gas having a smell by soaking an absorbent cotton with a liquid sample, instead of bubbling the liquid sample. The other one is that a three-way valve, instead of a check valve, is placed between the sample container and the outflow path.

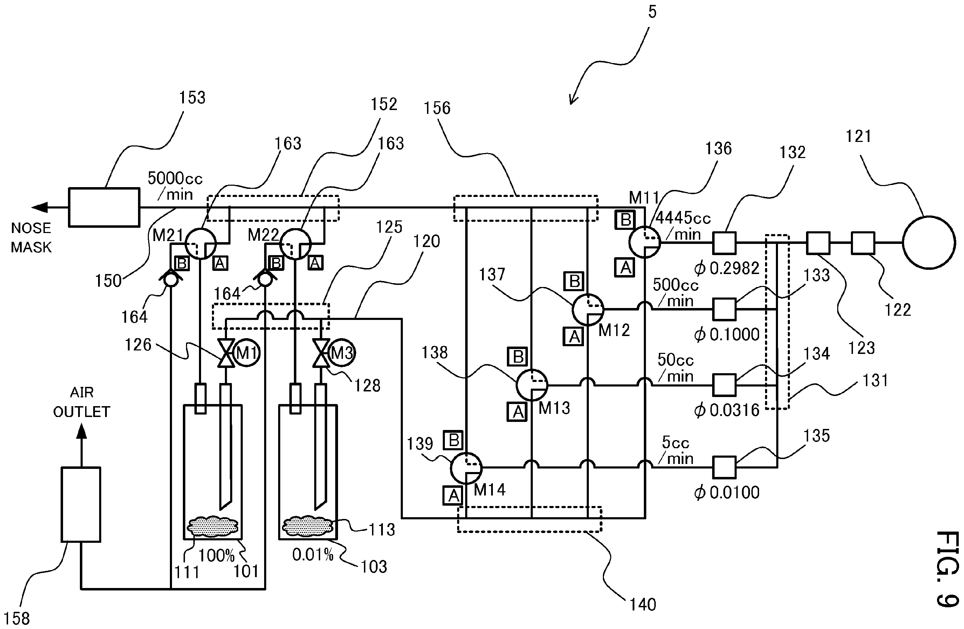

FIG. 9 is a block diagram illustrating a configuration of the olfaction test device 5 according to the present embodiment. As illustrated in FIG. 9, the olfaction test device 5 includes: a sample container 101 that contains an absorbent cotton 111 soaked with a 100% concentration of a liquid sample constituting a smell source; a sample container 103 that contains an absorbent cotton 113 soaked with a 0.01% concentration of a liquid sample constituting a smell source; an inflow path 120 that introduces air into the sample containers 101, 103; a three-way valve 163 that selects a destination of the gas forced to move out of the sample containers 101, 103 by the air flowing from the inflow path 120; an outflow path 150 that takes in and brings out the gas when the three-way valve 163 selects the direction to the nose mask as the destination of the gas; and an activated carbon filter 158 that removes smell components from the gas when the three-way valve 163 selects the direction to an air outlet as the destination of the gas.

The present embodiment is the same as Embodiment 3 regarding the configuration of the inflow path 120 from the compressor 121 to the inflow valves (M1, M3) 126, 128 and of the outflow path 150 from the heater 153 to the end. As described above, the present embodiment is different from Embodiment 3 in that the sample container is filled with a gas having a smell by soaking an absorbent cotton with a liquid sample instead of bubbling the liquid sample, and that a three-way valve, instead of a check valve, is placed between the sample container and the outflow path. The configuration of the control circuit 180 and its peripheral is also the same as in Embodiment 3, but the olfaction test process executed by the information terminal 184 is partly different. The information terminal 184 carries out an additional olfaction test preparation process, which is described later.

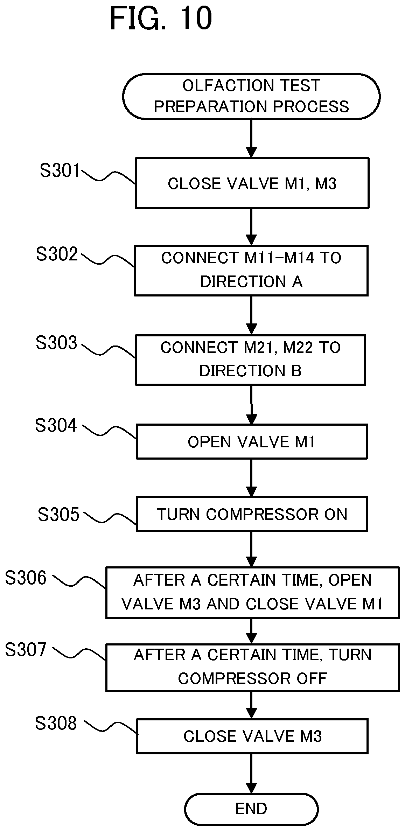

Initially, the sample containers 101, 103 are not filled with a smell of a liquid sample. Thus, a process for filling the sample containers 101, 103 with a smell (an olfaction test preparation process) is carried out prior to the olfaction test. The information terminal 184 includes a preparation start switch (not illustrated) that instructs the device to start the olfaction test preparation process. When the preparation start switch is pressed, the olfaction test preparation process is started. Referring to FIG. 10, the following describes the olfaction test preparation process.

First, the control circuit 180 closes all the inflow valves (M1 and M3) 126 and 128 (step S301). The control circuit 180 then connects all the three-way valves (M11, M12, M13, and M14) 136, 137, 138, and 139 to the direction A (step S302), and connects all the three-way valves (M21 and M22) 163 to the direction B (step S303).

Next, the control circuit 180 opens the inflow valve (M1) 126 (step S304), and then starts driving the compressor 121 (step S305). As the compressor 121 is driven, air starts flowing into the sample container 101 via the inflow path 120 and the inflow valve (M1) 126. The gas discharged from the sample container 101 passes through the three-way valve 163, the check valve 164, and the activated carbon filter 158 to be released. As a result, the sample container 101 becomes filled with a smell of the liquid sample at a concentration of 100% after a certain period of time.

Next, the control circuit 180 opens the inflow valve (M3) 128 and closes the inflow valve (M1) 126 (step S306). The air starts flowing into the sample container 103 via the inflow path 120 and the inflow valve (M3) 128. The gas discharged from the sample container 103 passes through the three-way valve 163, the check valve 164, and the activated carbon filter 158 to be released. As a result, the sample container 103 becomes filled with a smell of the liquid sample at a concentration of 0.01% after a certain period of time.

As the sample containers 101 and 103 are now filled with the respective smells, the control circuit 180 turns off the compressor 121 (step S307). The control circuit 180 then closes the inflow valve (M3) 128 (step S308) and exits the process.

Each of the sample containers 101 and 103 is now filled with a smell and thus is ready for the olfaction test.

As with the olfaction test device 3 according to Embodiment 3, the olfaction test device 5 changes the flow rate of the air flowing into the sample container 101 or 103 in accordance with the table in FIG. 7, instead of carrying out steps S104 to S108 in the olfaction test process illustrated in FIG. 3. In the present embodiment, connections of the three-way valve are changed depending on the corresponding inflow valve that is opened. Specifically, when the inflow valve (M1) 126 is opened (the inflow valve (M3) 128 is closed), the three-way valve (M21) 163 is connected to the direction A while the three-way valve (M22) 163 is connected to the direction B. When the inflow valve (M3) 128 is opened (the inflow valve (M1) 126 is closed), the three-way valve (M21) 163 is connected to the direction B while the three-way valve (M22) 163 is connected to the direction A.

Changing connections of the three-way valve 163 as above provides an effect similar to that of a check valve (the effect of preventing backflow of the gas having a smell into another sample container), thus eliminating the need for a check valve. In addition, gases of various concentrations as listed in the table in FIG. 7 can be released, which makes it possible to find at which gas concentration level, or smell intensity, the test subject can sense a smell.

As described above, the olfaction test device 5 according to the present embodiment causes the air flowing from the compressor 121 to divide into branches in the branching part 131 and to pass through the sonic nozzles 132, 133, 134, and 135 having different diameters. Then, the air passes through the three-way valves (M11, M12, M13, and M14) 136, 137, 138, and 139 disposed downstream of their corresponding sonic nozzles 132, 133, 134, and 135 to be either directed to the sample containers 101 and 103 or directly flowed into the nose mask 155, thereby producing gases in varying concentrations and accordingly with varying smell intensities. As a result, various gas concentration levels can be set to achieve a more accurate olfaction test.

In the olfaction test device 3 according to Embodiment 3, the gas discharged from the sample containers 101, 103 is to pass through the check valve 151. However, the gas flowing at a flow velocity lower than a certain value (for example, 100 cc/min) may result in a phenomenon in which the check valve 151 cannot be opened and the gas fails to flow into the outflow path 150. In the present embodiment, the three-way valve 163 is used instead of the check valve 151, enabling the gas to flow into the outflow path even when the gas is flowing at a lower flow velocity. As a result, the flow velocity of the gas can be made much lower, achieving an accurate olfaction test even when the gas concentration is much lower.

### (Embodiment 6)

Embodiment 6 of the present disclosure will now be described in detail with reference to the drawings.

As in Embodiments 1 to 5, an olfaction test device 6 according to the present embodiment flows a gas having a smell toward a test subject to determine whether the test subject senses the smell.

The olfaction test device 6 according to the present embodiment is made by changing the olfaction test device 4 according to Embodiment 4 such that, as with the olfaction test device 5 according to Embodiment 5, the sample container is filled with a gas having a smell by soaking an absorbent cotton with a liquid sample instead of bubbling the liquid sample, and a three-way valve, instead of a check valve, is placed between the sample container and the outflow path.

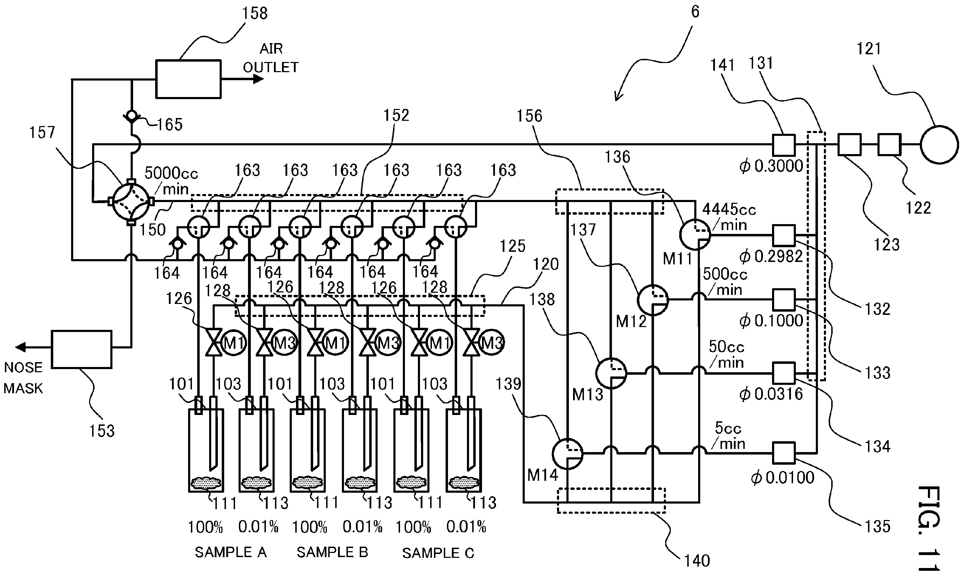

FIG. 11 is a block diagram illustrating a configuration of the olfaction test device 6 according to the present embodiment. As illustrated in FIG. 11, the olfaction test device 6 includes a plurality of pairs of sample containers 101 and 103, the sample container 101 containing an absorbent cotton 111 soaked with a liquid sample constituting a smell source at a concentration of 100%, and the sample container 103 containing an absorbent cotton 113 soaked with a liquid sample constituting a smell source at a concentration of 0.01%, where the type of liquid sample differs among the pairs. The olfaction test device 6 further includes: an inflow path 120 that introduces air into the sample containers 101, 103; a three-way valve 163 that selects a destination of the gas forced to move out of the sample containers 101, 103 by the air flowing from the inflow path 120; an outflow path 150 that takes in and brings out the gas when the three-way valve 163 selects the direction to the nose mask as the destination of the gas; and an activated carbon filter 158 that removes smell components from the gas when the three-way valve 163 selects the direction to an air outlet as the destination of the gas.

The present embodiment is the same as Embodiment 4 regarding the configuration of the inflow path 120 from the compressor 121 to the inflow valves (M1, M3) 126, 128 and of the outflow path 150 from the heater 153 to the end. As described above, the present embodiment is different from Embodiment 4 in that the sample container is filled with a gas having a smell by soaking an absorbent cotton with a liquid sample instead of bubbling the liquid sample, and that a three-way valve, instead of a check valve, is placed between the sample container and the outflow path. The present embodiment is also different from Embodiment 4 in that a check valve 165 is included so as to prevent backflow of the discharged air into a flush line during an olfaction test preparation process, because the activated carbon filter 158 is used both when the flush line is used and when the olfaction test preparation process is carried out.

Components of the olfaction test device covering from the inflow valves (M1, M3) 126, 128 to the check valve 164 are the same as those in Embodiment 5 except that the olfaction test device includes a plurality of inflow paths 120 led from the branching part 125 and inserted into the sample containers containing different types of liquid samples. In the present embodiment, for each of samples A, B, and C, the sample container 101 contains the absorbent cotton 111 soaked with a sample at a concentration of 100% while the sample container 103 contains the absorbent cotton 113 soaked with a sample at a concentration of 0.01%. The configuration of the control circuit 180 and its peripheral is also the same as in Embodiment 5. The olfaction test process executed by the information terminal 184 is partly different. The present embodiment is the same as Embodiment 5 in that the olfaction test preparation process is carried out prior to the olfaction test.

As in Embodiment 4, the air flowing out of the four sonic nozzles 132 to 135 and is directed to the joint 140 through the three-way valves (M11 to M14) 136 to 139 is combined in the joint 140. The combined air then passes through one opened valve among the six inflow valves 126 and 128 opposed to the sample containers containing the samples A, B, and C, and flows into one of the sample containers.

As in Embodiment 4, the inflowing air has a flow rate selected from the four different flow rates. Thus, the three types of liquid samples each having two concentration levels can emit gases having smells with the air flowing at four different flow rates, and accordingly, three types of smells each having eight different intensities can be produced.

As in Embodiment 4, a flush line is provided in the present embodiment, making it possible to clean the heater 153 and others with the flush line while smells are produced with different types of samples. As a result, mixing different smells from different types of samples can be avoided.

As described above, in the present embodiment, different types of smells each having a plurality of smell intensities are generated by causing air to flow at a predetermined flow rate through the sonic nozzles 132 to 135 and the three-way valves (M11 to M14) 136 to 139 into the sample containers 101, 103 containing different types of liquid samples. In addition, when smells are not produced, the air passing through the sonic nozzle 141 is caused to pass through the heater 153, the flexible hose 154, and the nose mask 155 via the four-way valve 157. As a result, different types of smells each having a plurality of intensities can be released from the nose mask 155 while the nose mask 155 can be cleaned with smell-free air, thereby achieving an accurate olfaction test including testing of the sense of different types of smells.

In the olfaction test device 4 according to Embodiment 4, the gas discharged from the sample containers 101, 103 is to pass through the check valve 151. However, the gas flowing at a flow velocity lower than a certain value (for example, 100 cc/min) may result in a phenomenon in which the check valve 151 cannot be opened and the gas fails to flow into the outflow path 150. In the present embodiment, the three-way valve 163 is used instead of the check valve 151, enabling the gas to flow into the outflow path even when the gas is flowing at a lower flow velocity. As a result, the flow velocity of the gas can be made much lower, achieving an accurate olfaction test even when the gas concentration is much lower.

### (Embodiment 7)

Embodiment 7 of the present disclosure will now be described in detail with reference to the drawings.

As in Embodiments 1 to 6, an olfaction test device 7 according to the present embodiment flows a gas having a smell toward a test subject to determine whether the test subject senses the smell.

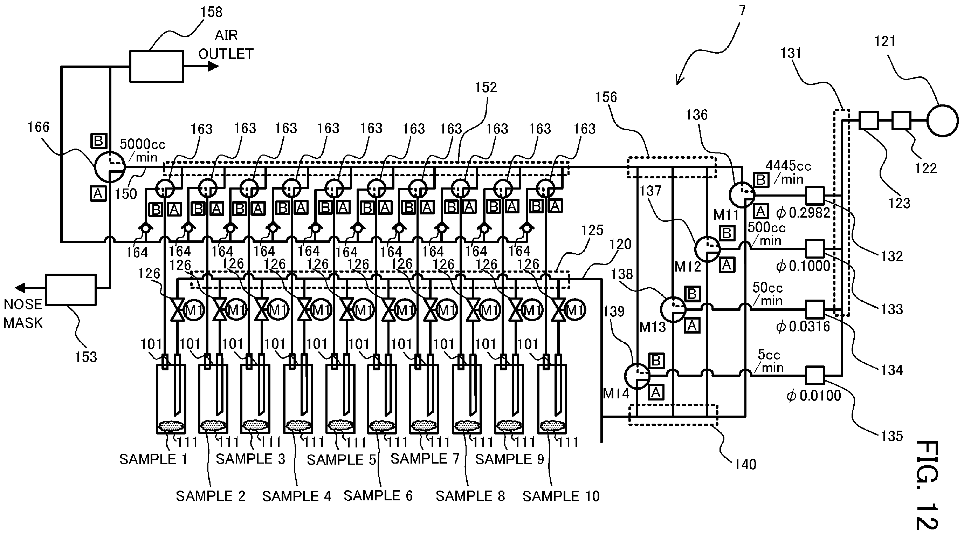

FIG. 12 is a block diagram illustrating a configuration of the olfaction test device 7 according to the present embodiment. As illustrated in FIG. 12, the olfaction test device 7 includes a plurality of sample containers 101 each containing an absorbent cotton 111 soaked with a liquid sample constituting a smell source, where the type of liquid sample differs among the sample containers. The olfaction test device 7 further includes: an inflow path 120 that introduces air into the sample containers 101; a three-way valve 163 that determines a destination of the gas forced to move out of the sample containers 101 by the air flowing from the inflow path 120, the destination selected from the outflow path 150 side and the check valve 164 side; an outflow path 150 that takes in and brings out the gas when the three-way valve 163 selects the direction to the outflow path 150 as the destination of the gas; an activated carbon filter 158 that removes smell components from the gas when the three-way valve 163 selects the direction to the check valve 164 as the destination of the gas; and a three-way valve 166 that selects the destination of the outflow path 150 from the nose mask side and the air outlet side.

The present embodiment is the same as Embodiment 6 in terms of components of the device, but is different in that each absorbent cotton 111 is soaked with a liquid sample whose concentration is fixed to a single value (usually, a liquid sample at a concentration of 100% is used), and that the smell intensity is varied through the three-way valves (M11 to M14) 136 to 139. The configuration of the control circuit 180 and its peripheral is also the same as in Embodiment 6, but the olfaction test process executed by the information terminal 184 is partly different. The information terminal 184 carries out an additional olfaction test preparation process, which is described later.

In the example in FIG. 12, only four three-way valves (M11 to M14) 136 to 139 are disposed, but other sonic nozzles may be added, such as four other sonic nozzles having much smaller diameters (corresponding air flow rates are 0.5 cc/min, 0.05 cc/min, 0.005 cc/min, and 0.0005 cc/min). In this case, gas concentrations ranging from 100% to 0.00001% are made selectable by choosing an air flow rate appropriately.

The present embodiment is different from Embodiments 4 and 6 in that a three-way valve 166 is included instead of the four-way valve 157. In Embodiments 4 and 6, the heater 153 and others can be cleaned with the flush line that includes the sonic nozzle 141 and the four-way valve 157. In the present embodiment, the outflow path 150 can be used as the flush line by causing all the three-way valves (M11 to M14) 136 to 139 to be connected to the joint 156 and all the three-way valves 163 to be connected to the check valves 164. This can avoid mixing different smells from different types of liquid samples by using the outflow path 150 as the flush line without using the four-way valve 157, which is more expensive.

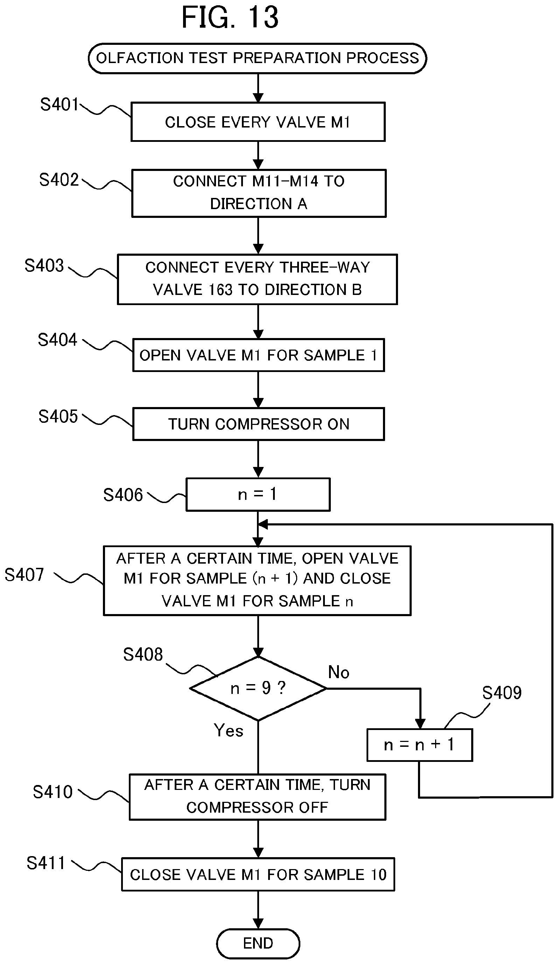

As in the foregoing embodiments, each of the sample containers 101 of the present embodiment is not initially filled with a smell of a liquid sample. Thus, a process for filling the sample container 101 with a smell (an olfaction test preparation process) is carried out prior to the olfaction test. The information terminal 184 includes a preparation start switch (not illustrated) that instructs the device to start the olfaction test preparation process. When the preparation start switch is pressed, the olfaction test preparation process is started. Referring to FIG. 13, the following describes the olfaction test preparation process.

First, the control circuit 180 closes all the inflow valves (M1) 126 (step S401). The control circuit 180 then connects all the three-way valves (M11, M12, M13, and M14) 136, 137, 138, and 139 to the direction A (step S402), and connects all the three-way valves 163 to the direction B (step S403).

Next, the control circuit 180 opens the inflow valve (M1) 126 that is connected to the sample container 101 containing the sample 1 (step S404), and then starts driving the compressor 121 (step S405). As the compressor 121 is driven, air starts flowing into the sample container 101 containing the sample 1 via the inflow path 120 and the inflow valve (M1) 126 that is connected to the sample container 101 containing the sample 1. The gas discharged from the sample container 101 passes through the three-way valve 163, the check valve 164, and the activated carbon filter 158 to be released. As a result, the sample container 101 containing the sample 1 becomes filled with a smell of the sample 1 after a certain period of time.

In order that the sample container 101 containing the sample n is filled with a smell of the sample n, the control circuit 180 initially sets the variable n to 1 (step S406). After a certain period of time, the control circuit 180 opens the inflow valve (M1) 126 connected to the sample container 101 containing the sample (n + 1) and closes the inflow valve (M1) 126 connected to the sample container 101 containing the sample n (step S407). The air starts flowing into the sample container 101 containing the sample (n + 1) via the inflow path 120 and the inflow valve (M1) 126 connected to the sample container 101 containing the sample (n + 1). The gas discharged from the sample container 101 containing the sample (n + 1) passes through the three-way valve 163, the check valve 164, and the activated carbon filter 158 to be released. As a result, the sample container 101 containing the sample (n + 1) becomes filled with a smell of the sample (n + 1) after a certain period of time.

Next, the control circuit 180 determines whether n is equal to 9 (step S408). If n is not equal to 9 (No in step S408), the control circuit 180 adds 1 to n (step S409) and returns to step S407. If n is equal to 9 (Yes in step S408), the control circuit 180 turns off the compressor 121 after a certain period of time (step S410). The control circuit 180 then closes the inflow valve (M1) 126 that is connected to the sample container 101 containing the sample 10 (step S411) and exits the process.

Each of the sample containers 101 containing the sample n (where n = 1 to 10) is now filled with a smell of the sample and thus is ready for the olfaction test.

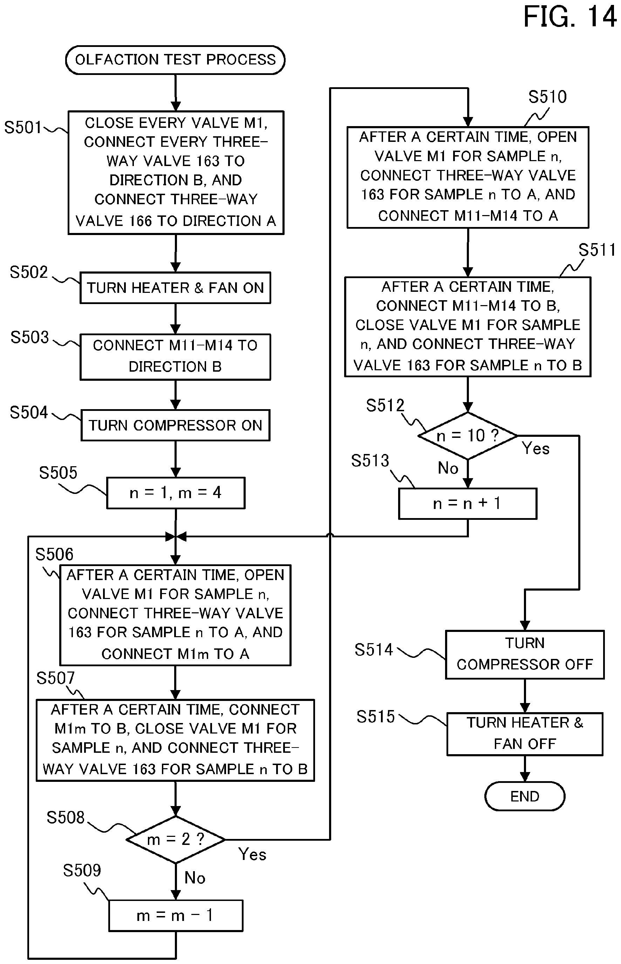

Referring to FIG. 14, the following describes the olfaction test process carried out by the olfaction test device 7. The olfaction test process starts when the main power switch 181 is turned on and the information terminal 184 executes the olfaction test program.

The control circuit 180 begins with initializing the individual valves by closing all the inflow valves (M1) 126, connecting all the three-way valves 163 to the direction B, and connecting the three-way valve 166 to the direction A (step S501). The control circuit 180 then supplies power to, and starts driving, the heater 153 and the fan 162 (step S502). Next, the control circuit 180 connects all the three-way valves (M11 to M14) 136 to 139 to the direction B (step S503), and then starts driving the compressor 121 (step S504). As the compressor 121 is driven, air starts flowing into the outflow path 150.

Upon start of the olfaction test, the control circuit 180 sets the variable n to 1 and the variable m to 4 (step S505) and, after a certain period of time, opens the inflow valve (M1) 126 that is connected to the sample container 101 containing the sample n and connects the three-way valve 163 that is connected to the sample container 101 containing the sample n to the direction A, and then connects the three-way valve (M1m) to the direction A (step S506). For example, in the case of m = 4, the three-way valve (M14) 139 is connected to the direction A. As a result, the air that flows out of the sonic nozzles 132 to 135 and passes through one of the three-way valves (M11 to M14) 136 to 139 that is connected to the direction A is released into the sample container 101 containing the sample n. The former step in step S506 (opening the inflow valve (M1) 126) is called a switching step where the control circuit 180 functions as a switcher. The latter step in step S506 (connecting the three-way valve (M1m) to the direction A) is called an inflow step where the control circuit 180 functions as an inflow controller.

Since the sample container 101 is filled with a smell component of the sample n, the gas in a volume equal to that of the air released into the sample container 101 is discharged from the sample container 101 to the outflow path 150. Then, the gas having a smell at a concentration resulting from mixing the gas discharged from the sample container 101 with the air that has passed through any of the three-way valves (M11 to M14) 136 to 139 that is connected to the direction B goes through the outflow path 150, the three-way valve 166, and the heater 153 to reach the nose mask. Upon sensing the smell of the gas, the test subject presses the sense switch 182. The control circuit 180 sends to the information terminal 184 a signal indicating that the sense switch 182 has been pressed, and the information terminal 184 stores the result indicating that a smell has been sensed.

Note that the gas is warmed by the heater 153 to a temperature falling within a predetermined range before released into the nose mask 155 because a smell is sensed differently depending on the temperature. The air flowing out of the sonic nozzles 132 to 135 flows at a constant flow velocity and a constant flow rate, and is finally combined in the outflow path 150 after passing through either direction A or B directed at the three-way valves (M11 to M14) 136 to 139. Therefore, the gas flowing from the outflow path 150 to the nose mask also flows at a constant flow velocity and a constant flow rate. The gas flows without interruption because the inflow valve (M1) 126 connected to the sample container 101 containing the sample (n + 1) is opened and then the inflow valve (M1) 126 connected to the sample container 101 containing the sample n is closed. Therefore, the test subject is prevented from determining, attributable to a change in flow velocity or flow rate of the gas, that the smell has changed.

When a certain period of time has passed after connection of the three-way valve (M1m) to the direction A, the control circuit 180 connects the three-way valve (M1m) to the direction B, and then closes the inflow valve (M1) 126 connected to the sample container 101 containing the sample n, and connects the three-way valve 163 that is connected to the sample container 101 containing the sample n to the direction B (step S507). As a result, all the air flowing out of the sonic nozzles 132 to 135 is directed to the outflow path 150, and the smell-free air passes through the outflow path 150 to be released from the nose mask 155.

Then, the control circuit 180 determines whether m is equal to 2 (step S508). If m is not equal to 2 (No in step S508), the control circuit 180 subtracts 1 from m (step S509) and returns to step S506. The control circuit 180 thereafter conducts the olfaction test with regard to the sample n while increasing the concentration by repeating steps S506 and S507.

When it is determined that m is equal to 2 in step S508 (Yes in step S508), the control circuit 180 directs the air flowing out of all the sonic nozzles 132 to 135 to the sample container containing the sample n. Specifically, after a certain period of time, the control circuit 180 opens the inflow valve (M1) 126 connected to the sample container 101 containing the sample n and connects the three-way valve 163 connected to the sample container 101 containing the sample n to the direction A, and then connects all the three-way valves (M11 to M14) 136 to 139 to the direction A (step S510). As a result, all the air that flows out of the sonic nozzles 132 to 135 is released into the sample container 101 containing the sample n.

After a certain period of time, the control circuit 180 connects all the three-way valves (M11 to M14) 136 to 139 to the direction B, and then closes the inflow valve (M1) 126 connected to the sample container 101 containing the sample n, and connects the three-way valve 163 that is connected to the sample container 101 containing the sample n to the direction B (step S511).

The olfaction test has now been conducted with regard to the sample n at the different concentrations. Then, the control circuit 180 determines whether n is equal to 10 (step S512). If n is not equal to 10 (No in step S512), the control circuit 180 adds 1 to n (step S513) and returns to step S506. If n is equal to 10 (Yes in step S512), the control circuit 180 turns off the compressor 121 (step S514). Next, the heater 153 and the fan 162 being driven are stopped (step S515), and the process is exited.

In this way, the air is caused to flow out of the sonic nozzles 132 to 135 at a constant flow velocity and a constant flow rate, while the air in part or in whole is successively caused to flow into the sample containers 101 containing ten difference samples, whereby the gas having a smell at different intensities (and different types of smells) is released from the nose mask 155. The test subject inhales gases with different smell intensities (and different types of smells) at regular time intervals to be tested whether the test subject can sense the smells.

In the olfaction test process illustrated in FIG. 14, a lowest smell intensity is gradually increased to a highest smell intensity for one sample, which is then switched to another sample and the same steps are repeated. However, the test is not limited to this order. For example, the smell intensity may be initially fixed to a highest one (m = 1) and the test may be conducted for each sample at this smell intensity. After the test is conducted with regard to all the samples at this intensity, the test may be done at a lower smell intensity. In the present embodiment, an absorbent cotton is soaked with a liquid sample and contained in a sample container. However, the material soaked with a liquid sample is not limited to absorbent cottons, and any material may be used as long as the material can be soaked with a certain amount of a liquid sample.

As described above, in the present embodiment, different types of smells each having a plurality of smell intensities are generated by causing air to flow at a predetermined flow rate through the sonic nozzles 132 to 135 and the three-way valves (M11 to M14) 136 to 139 into the sample containers 101 containing different types of liquid samples. When no smell is being created, all the three-way valves (M11 to M14) 136 to 139 are connected to the joint 156 side and all the three-way valves 163 are connected to the check valve 164 side so that the outflow path 150 serves as a flush line, and the air passing through the three-way valves (M11 to M14) 136 to 139 goes through the three-way valve 166, the heater 153, the flexible hose 154, and the nose mask 155. As a result, different types of smells each having a plurality of intensities can be released from the nose mask 155 while the nose mask 155 can be cleaned with smell-free air, thereby achieving an accurate olfaction test including testing of the sense of different types of smells.

In the present embodiment, the three-way valve 163 is used instead of the check valve 151, enabling the gas to flow into the outflow path even when the gas is flowing at a lower flow velocity. As a result, the flow velocity of the gas can be made much lower, achieving an accurate olfaction test even when the gas concentration is much lower.

In the foregoing embodiments, the flow rate of the air output from the compressor 121 is controlled by using the sonic nozzles 124, 132 to 135, and 141, but sonic nozzles may not necessarily be used for controlling the flow rate. In any of the foregoing embodiments, the sonic nozzles can be replaced by flow rate control devices such as a mass flow controller or flow rate control valves such as a throttle valve. For example, using a mass flow controller instead of a sonic nozzle is advantageous in that flow rates can be easily adjusted, although mass flow controllers are more expensive. Flow rate control devices such as sonic nozzles and mass flow controllers and flow rate control valves such as throttle valves each function as a flow rate controller controlling the flow rate of the air output from the compressor 121.

As seen above, in the olfaction test device according to the present disclosure, a sample container contains an absorbent cotton soaked with a liquid sample constituting a smell source or contains the liquid sample itself, the air flowing at a constant flow velocity and at a predetermined flow rate is introduced into the sample container or into the liquid sample therein, the inflowing air is passed or shut off at a point midway in the inflow path, the gas forced to move out by the air flowing from the inflow path or the gas generated by bubbling the sample with the air flowing from the inflow path is taken into the outflow path, and the gas is released toward the test subject. As a result, an accurate test can be conducted without causing the test subject to sense a change in smell based on other factors. The present disclosure has solved the problem of insufficient accuracy of olfaction tests posed by conventional techniques, making it possible to provide a more accurate olfaction test.

The foregoing describes some example embodiments for explanatory purposes. Although the foregoing discussion has presented specific embodiments, persons skilled in the art will recognize that changes may be made in form and detail without departing from the broader spirit and scope of the invention. Accordingly, the specification and drawings are to be regarded in an illustrative rather than a restrictive sense. This detailed description, therefore, is not to be taken in a limiting sense, and the scope of the invention is defined only by the included claims, along with the full range of equivalents to which such claims are entitled.

For example, Embodiments 1 and 2 described above are configured such that the test is conducted with a single type of liquid sample at different gas concentrations. However, different types of liquid samples may be used to generate different types of smells as in Embodiments 4, 6, and 7. In this case, sample containers 101 and 103 containing liquid samples at different concentrations may be prepared for each of two or more types of liquid samples, and these containers may be connected to the branching part 125 via the inflow valves 126 and 128. As in Embodiments 4 and 6, the olfaction test devices in Embodiments 1 and 2 each may include a flush line that includes another sonic nozzle 141 and the four-way valve 157. As in Embodiment 7, the olfaction test devices in Embodiments 1 to 6 each may include a flush line that includes the outflow path 150 and the three-way valve 166.

In the foregoing embodiments, a liquid sample has concentrations of 100%, 1%, and 0.01% and the gas has a total flow rate of 5,000 cc/min. However, numerical values to be selected are not limited thereto and any combination of numerical values suitable for the test may be selected. In addition, flow rates and concentrations of the gas may be changed as appropriate depending on the type of the sample and the purpose of the test.

In the foregoing embodiments, the olfaction test process is carried out by executing a program installed on the information terminal 184. However, a general computer may execute the program for the process executed by the information terminal 184, whereby an apparatus including the computer can function as any of the olfaction test devices 1 to 7 according to the present disclosure.

Such program may be distributed by any method. For example, the program may be stored and distributed in a non-transitory computer-readable recording medium such as a compact disc read-only memory (CD-ROM), a digital versatile disc (DVD), a magneto-optical disc (MO), or a memory card, or may be distributed via communication networks such as the Internet.

This application claims the benefit of Japanese Patent Application No. 2017-39789, filed on March 2, 2017, and Japanese Patent Application No. 2018-29497, filed on February 22, 2018, of which the entirety of the disclosures is incorporated by reference herein.

### Reference Signs List

- 1, 2, 3, 4, 5, 6, 7: Olfaction test device
- 101 to 104: Sample container
- 120: Inflow path
- 121: Compressor
- 122: Pressure reducing valve
- 123: Filter
- 124, 132 to 135, 141: Sonic nozzle
- 125, 131: Branching part
- 126 to 129: Inflow valve
- 130, 136 to 139, 163, 166: Three-way valve
- 140, 152, 156: Joint
- 150: Outflow path
- 151, 164, 165: Check valve
- 153: Heater
- 154: Flexible hose
- 155: Nose mask
- 157: Four-way valve
- 160: Supporting platform
- 158, 161: Activated carbon filter
- 162: Fan
- 180: Control circuit
- 181: Main power switch
- 182: Sense switch
- 183: USB connector
- 184: Information terminal

## Claims

1. An olfaction test device comprising:
a sample container that contains a liquid sample constituting a smell source;
an inflow path that causes air having a predetermined flow rate to flow into the sample container at a constant flow velocity;
an inflow valve that passes or shuts off the inflowing air at a point in the inflow path; and
an outflow path that takes in, and flows out toward a test subject, a gas discharged from the sample container by the air inflowing from the inflow path.

2. The olfaction test device according to claim 1, further comprising:
a compressor that flows air having at least a certain barometric pressure into the inflow path; and
a flow rate controller that is inserted into the inflow path upstream of the inflow valve.

3. The olfaction test device according to claim 1, further comprising:
a compressor that flows air having at least a certain barometric pressure into the inflow path;
a first branching part that divides an output of the compressor into branches;
flow rate controllers whose number is equal to the number of the branches, the flow rate controllers passing the individual branches of the air divided by the first branching part and having different diameters from one another; and
a first joint that joins together the gas that has been discharged from the sample container by causing at least part of the air passing through the individual flow rate controllers to flow into the sample container and the air that has passed the individual flow rate controllers but has not been caused to flow into the sample container.

4. The olfaction test device according to claim 2 or 3, further comprising:
a three-way valve including an input end, one output end, and another output end, the input end being connected to an output of the flow rate controller, the one output end being connected to the inflow valve, and the other output end being connected to the outflow path,
wherein the air that has passed through the flow rate controller is switchable between being directed to the inflow valve side and being directed to the outflow path side.

5. The olfaction test device according to any one of claims 1 to 4, comprising:
the sample containers, the number of which is two or more, the individual sample containers containing the liquid samples of different types or at different concentrations from one another; and
the inflow valves, the number of which is equal to the number of the sample containers, the individual inflow valves passing or shutting off the air caused to flow into the individual sample containers,
wherein
the inflow path comprises a second branching part that divides the path into branches upstream of the inflow valves, the number of which branches is equal to the number of the sample containers, and
the outflow path flows the gas out, the gas having been discharged from one of the sample containers into which the air flows after passing through the inflow valve.

6. The olfaction test device according to any one of claims 1 to 5, further comprising:
a flush line that flows the air having a flow rate equal to the flow rate of the inflow path; and
a switching valve that switches the air flowing along the flush line between being flowed into the outflow path and being released outside.

7. The olfaction test device according to any one of claims 1 to 6, further comprising:
a heater that is disposed in the outflow path and warms the gas discharged from the sample container to a temperature falling within a predetermined range.

8. The olfaction test device according to any one of claims 1 to 7, wherein the outflow path comprises a check valve that passes the gas directed from the sample container to the test subject and shuts off the gas directed from the test subject to the sample container.

9. The olfaction test device according to any one of claims 1 to 7, further comprising:
a filter that removes a smell component from the gas discharged from the sample container; and
a three-way valve that switches the gas discharged from the sample container between being directed to the test subject and being directed to the filter.

10. An olfaction test method for releasing a gas generated from a liquid sample toward a test subject, the liquid sample being contained in a sample container and constituting a smell source, and for determining whether the test subject senses the smell of the gas, the method comprising:
an inflow step of causing air having a predetermined flow rate to inflow toward the sample container at a constant flow velocity; and
a switching step of switching between discharging and not discharging the gas having a smell from the sample container by switching an inflow valve that passes or shuts off the air that is caused to inflow in the inflow step.

11. A program executed by a computer for releasing a gas generated from a liquid sample toward a test subject, the liquid sample being contained in a sample container and constituting a smell source, and for determining whether the test subject senses the smell of the gas, the program causing the computer to function as:
an inflow controller that drives a compressor and causes air having a predetermined flow rate to inflow toward the sample container at a constant flow velocity; and
a switcher that switches between discharging and not discharging the gas having a smell from the sample container by switching an inflow valve that passes or shuts off the air that is controlled to inflow by the inflow controller.
